(19) Europäisches Patentamt
European Patent Office
Office européen des brevets

(11) **EP 2 912 472 B1**

(12) **EUROPEAN PATENT SPECIFICATION**

(45) Date of publication and mention of the grant of the patent:
**01.08.2018 Bulletin 2018/31**

(21) Application number: **13786604.2**

(22) Date of filing: **25.10.2013**

(51) Int Cl.:
***G01N 33/68*** (2006.01)

(86) International application number:
**PCT/US2013/066860**

(87) International publication number:
**WO 2014/066787 (01.05.2014 Gazette 2014/18)**

(54) **DIABETES PANEL**

DIABETES-PANEL

BILAN DIABÉTIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **26.10.2012 US 201213662113**

(43) Date of publication of application:
**02.09.2015 Bulletin 2015/36**

(73) Proprietor: **Boston Heart Diagnostics Corporation Framingham, MA 01702 (US)**

(72) Inventor: **SCHAEFER, Ernst J. Natick, MA 01760 (US)**

(74) Representative: **Kirkham, Nicholas Andrew et al Graham Watt & Co. LLP St. Botolph's House 7-9 St. Botolph's Road Sevenoaks, Kent TN13 3AJ (GB)**

(56) References cited:
**WO-A1-2006/072654     WO-A1-2007/128884
WO-A1-2011/058232     WO-A1-2013/078122**

• **S. P. JURASCHEK ET AL: "Alternative Markers of Hyperglycemia and Risk of Diabetes", DIABETES CARE, vol. 35, no. 11, 8 August 2012 (2012-08-08), pages 2265-2270, XP055095686, ISSN: 0149-5992, DOI: 10.2337/dc12-0787**

## Description

<u>Field of the Invention</u>

The present invention generally relates to predicting the risk of diabetes in patients who currently do not have the disease.

<u>Background</u>

Diabetes mellitus is a group of metabolic diseases characterized by high sugar levels in the blood, attributable to inadequate production of insulin by the body or to the body's failure to respond to the insulin that is produced. Diabetes mellitus has been linked to heart disease, stroke, kidney failure, neuropathy, and retinopathy.

Although several types of diabetes exist, the vast majority of cases involve Type 2 diabetes. Patients with Type 2 diabetes produce insulin, however, they do not respond to the insulin that is produced. As a result, these patients often have abnormally high levels of insulin in addition to elevated glucose levels.

The incidence of Type 2 diabetes has increased dramatically in recent years, reaching epidemic levels in at least the United States. According to one study conducted by the UnitedHealth Center for Health Reform & Modernization, more than half of all Americans may develop diabetes or "prediabetes" (elevated glucose levels that are below diabetes cut-offs) by the year 2020 unless proper measures are taken. In many cases, lifestyle changes such as weight loss, exercise, and informed eating habits can contribute significantly to the prevention of disease. In cases where medication is necessary, drug adherence earlier on can help stave off diabetes-related complications later in life.

Accordingly, the ability to accurately predict a patient's risk of developing diabetes is essential to disease prevention and management. An easy-to-use, accurate diabetes prediction test could focus patients on preventative measures, thereby staving off exorbitant medical costs in addition to mitigating the severity of disease. Many tests for diabetes only involve detecting existing diabetes in a patient and unfortunately, are not useful for predicting the development of diabetes in patients who are currently negative for the disease. One such test is the fasting blood glucose (FBG) test, which measures blood glucose levels after a fast. If levels are above a certain threshold, the patient is diagnosed as having diabetes. Although the FBG test is relatively simple and easy to administer, its ability to predict future diabetes as currently implemented is limited. For example, it is known that many people diagnosed as pre-diabetic using FBG never go on to develop diabetes.

Other tests are often used in conjunction with FBG to confirm the diagnosis of existing diabetes. These tests may include, for example, the oral glucose tolerance test (OGTT) and the Hemoglobin Alc (HbAlc) test. With OGTT, a patient is given a standard dose of glucose to ingest by mouth and blood levels are assessed usually two hours later. Due to these testing conditions, OGTT can be inconvenient for both doctors and patients alike. The HbAlc test measures the amount of glucose that is attached to hemoglobin in the blood, which provides an indication of what average blood sugar levels were during the previous two to three months.

A common thread with all these methods as conventionally implemented is that they are retrospective rather than forward-looking. In other words, they can only be used to detect existing diabetes in patient rather than predict the risk of diabetes development in patients that are currently non-diabetic. Recently, a number of panels for predicting diabetes have been developed, but even these panels indicate room for improvement, especially in terms of accuracy. Accordingly, there is a continued need for improved methods able to predict the risk of incident diabetes. Juraschek et al., (2012) discloses the results of a population study in which several parameters are measured in serum samples of healthy patients, taken at the beginning of the study, and are correlated with the development of diabetes during the study. Among the parameters that have a predictive value is percent glycated albumin of total serum albumin, fasting glucose, family history of diabetes, and BMI.

<u>Summary</u>

The present invention generally relates to predicting the risk of developing a diabetic condition. More specifically, the invention relates to methods (and associated panels) according to the claims. In contrast to conventional methods that only detect an existing diabetic condition (i.e., people who already have diabetes). The invention recognizes that the risk of developing diabetes in patients presenting with no clinical symptoms is linked to a variety of different parameters, including elevated levels of glycated albumin. Methods are described for predicting the risk of diabetes based upon determining a glycated albumin level in a patient sample. A panel for predicting the risk of diabetes that includes the testing of glycated albumin levels in a patient sample is also described. Levels beyond a predetermined threshold indicate a risk of developing a diabetic condition at a future time.

The invention also encompasses the use of additional parameters, alone or in combination, which are predictive of diabetes onset. These parameters include biomarkers obtained from patient samples, including elevated glucose levels, elevated adiponectin levels, and elevated triglyceride levels. A patient sample can be derived from urine, cerebrospinal fluid, seminal fluid, saliva, sputum, stool, tissue, or blood.

The invention also encompasses the use of biomarkers that are not necessarily obtained from a patient sample. Examples include a patient's body mass index (BMI), which may further indicate the onset of diabetes if above a predetermined threshold. Further examples include the patient's family history of diabetes, or if the patient has been treated with statins. The biomarkers encompassed by the invention are relatively simple to evaluate, unlike the markers tested with OGTT.

Using the methods (and associated panels) of the invention, the risk of developing diabetes is ascertained for patients who currently do not have the disease. Moreover, methods (and associated panels) of the invention can predict the onset of diabetes over a period of several years. For example, it has been found that methods (and associated panels) of the invention are useful to predict the onset of diabetes over, for example, an eight year period. In addition, it has also been found that the methods (and associated panels) of the invention provide greater predictive accuracy (i.e., specificity and sensitivity) than other diabetes predictive models known in the art. Accordingly, the methods (and associated panels) of the invention permit the improved prediction of developing diabetes, thereby facilitating the early implementation of measures to prevent or manage the disease.

Brief Description of the Drawings

FIG. 1 illustrates an exemplary system for performing methods of the invention.
FIG. 2 is an exemplary process chart depicting the process steps for predicting the risk of developing a diabetic condition.
FIG. 3 is a flow diagram of an exemplary method for developing a model which may be used to evaluate a risk of a person, or group of people, for developing a diabetic condition.
FIG. 4 is a flow diagram of an exemplary method for using a model to evaluate a risk of a subject (e.g., a person, or group of people) developing a diabetic condition.

Detailed Description

The invention generally relates to predicting the risk of developing a diabetic condition. More specifically, the invention relates to methods (and associated panels) according to the claims. Methods are described which can involve obtaining a sample from a patient who presents as negative for diabetes, and conducting an assay on the sample to obtain a level of glycated albumin. The method can further involve determining an elevated risk of developing a diabetic condition if the level exceeds a predetermined threshold. Panels of the invention can involve at least one assay for determining a level of glycated albumin in a patient sample obtained from a patient who does not have diabetes at the time the sample is obtained, in which the results from the assay indicate a risk of developing diabetes at a future time.

Methods and panels are described which are useful for all types of diabetic conditions. Diabetic conditions can include various types of diabetes mellitus, including autoimmune and idiopathic Type 1 diabetes mellitus and Type 2 diabetes mellitus. According to criteria established by the World Health Organization, diabetes mellitus is defined as having a fasting plasma glucose concentration greater than or equal to 7.0 mmol/l (126 mg/dl) or a 2-hour glucose level greater than equal to 11.1 mmol/l (200 mg/dl).

Diabetic conditions can also include pre-diabetes, which refers to a physiological state in which the patient's glucose levels are above normal, but not yet at established levels for diabetes. Specific categories of pre-diabetes or pre-diabetic conditions can include, for example, Impaired Glucose Tolerance (IGT), and Impaired Fasting Glycemia (IFG). IGT refers to post-prandial abnormalities of glucose regulation, while IFG refers to abnormalities that are measured in a fasting state. The World Health Organization defines values for IFG as a fasting plasma glucose concentration of 6.1 mmol/L (100 mg/dL) or greater, but less than 7.0 mmol/L (126 mg/dL). While there are some individuals diagnosed as pre-diabetic that will eventually develop Type 2 diabetes, many individuals with pre-diabetic conditions will not convert.

Accordingly, methods (and associated panels) of the invention are useful for evaluating the risk of developing a diabetic condition in subjects that currently do not have or are negative for diabetes.
In other words, these subjects are asymptomatic for the disease or do not yet meet the established criteria for diabetes mellitus at the time of testing. In certain aspects of the invention, the subject may initially be completely negative for any diabetic condition, including pre-diabetes. In this instance, the risk of developing a diabetic condition includes the risk of developing diabetes, pre-diabetes, or both pre-diabetes and diabetes. In other aspects of the invention, the subject may initially be negative for diabetes, but positive for a pre-diabetic condition. In this instance, the risk of developing a diabetic condition includes the risk of developing diabetes alone.

Risk refers to the probability that an event will occur over a specific time period, such as a conversion from a non-diabetic state to a state associated with a diabetic condition. In risk evaluation, a prediction is made regarding the likelihood that an event or disease state may occur, or the rate of occurrence of the event or conversion from one disease state to another. This can include, for example, conversion from a normal glycemic condition to pre-diabetes or diabetes, conversion from one pre-diabetic condition to another pre-diabetic condition, or conversion from a pre-diabetes to diabetes. The methods of the present invention may be used to make continuous or categorical measurements of the risk of conversion to Type 2 diabetes, thus diagnosing and defining the risk spectrum of a category of subjects defined as pre-diabetic. In the categorical scenario, the invention can be used to discriminate between normal and pre-diabetes subject cohorts. In other embodiments, the present invention may be used so as to discriminate pre-diabetes from diabetes, or diabetes from normal. Such differing use may require different parameter combinations in individual panels, mathematical algorithm, and/or cut-off points, but be subject to the same aforementioned measurements of accuracy for the intended use.

Methods (and associated panels) of the invention contemplate the use of patient-derived samples that are used in assays or tests in order to obtain particular information. Samples generally refer to biological samples isolated from a subject and can include, without limitation, whole blood, serum, plasma, blood cells, endothelial cells, tissue biopsies, lymphatic fluid, ascites fluid, interstitital fluid (also known as "extracellular fluid" and encompasses the fluid found in spaces between cells, including, inter alia, gingival crevicular fluid), bone marrow, cerebrospinal fluid (CSF), saliva, mucous, sputum, sweat, urine, or any other secretion, excretion, or other bodily fluids. In preferred embodiments, the patient sample is a blood sample, which can include whole blood or any fraction thereof, including blood cells, serum and plasma. The information obtained relates to certain biomarkers that are evaluated when determining the risk of developing a diabetic condition. Certain biomarkers can be accessed through the patient sample. These biomarkers can refer to naturally occurring molecules, genes, or characteristics that can be used to monitor a physiological process or condition. Biomarkers suitable for use with the invention include, without limitation, proteins, nucleic acids, and metabolites, together with their polymorphisms, mutations, variants, modifications, subunits, fragments, protein-ligand complexes, and degradation products, protein-ligand complexes, elements, related metabolites, and other analytes or sample-derived measures. Further exemplary biomarkers obtainable from patient samples include without limitation, glycated albumin levels, fasting glucose levels, adiponectin levels, and triglyceride levels. These specific biomarkers can be used alone, or in any combination with each other.

The invention also contemplates the assessment of biomarkers that are not typically associated with patient derived samples. Such biomarkers would include the dietary or eating habits of the patient. Another would be the exercise habits of the patient. Further exemplary biomarkers of the invention not associated with patient samples include, without limitation, the body mass index of the patient, the family history of the patient regarding diabetes, and whether or not the patient has received statin therapy. Much of the information related to these characteristic biomarkers can be obtained simply by asking the patient the appropriate questions. These specific biomarkers can be used alone, or in any combination with each other. In addition, these non-sample-derived biomarkers can be further combined with the sample-derived biomarkers discussed above.

Further detail will now be provided on particular biomarkers obtained from patient-derived samples, including glycated albumin levels, fasting glucose levels, adiponectin levels, and triglyceride levels. The incorporation of these biomarkers into the encompassed methods and panels assist in the evaluation of risk for developing a diabetic condition.

Glycated albumin refers to the amount of glucose that collects on the protein albumin. Albumin is the largest component of plasma proteins, representing more than 80% of the total proteins and 60% of the total plasma protein concentration. The use of glycated albumin in the detection of existing diabetes is well-known in the art. It has been found, however, that glycated albumin levels can also be used to predict the development of future diabetes in patients who are currently negative for the disease. Accordingly, there are described methods and panels that encompass conducting an assay on a patient sample to obtain a level of glycated albumin and comparing that level to a threshold level of glycated albumin. A level of glycated albumin exceeding the predetermined threshold indicates a risk of developing a diabetic condition.

Any method known in the art is useful for the isolation and quantification of glycated albumin. These include, but are not limited to, enzymatic assay, high-performance liquid chromatography (HPLC) and affinity chromatography, immunoassay, including quantification by radioassay, enzyme-linked immunosorbent assay (ELISA), colorimetry, and electrochemical assays. These techniques are well-known in the art, and are discussed hereinafter. Assays for detecting glycated albumin include forming a complex with glycated albumin present in a sample and capture molecules that have been introduced into the sample. The formed complexes can be detected (via a fluorescent label, qPCR, sequencing, enzymatically), thereby allowing detection of the glycated albumin. Further detail regarding the quantification of glycated albumin can be found in U.S. Patent Nos. 7,659,107 and 7,871,789 as well as U.S. Patent Appl. Nos. 2007/0015291, 2009/0246801, and 2010/0167306.

In some embodiments of the invention, a level of glycated albumin greater than or equal to 3% of total albumin indicates a risk of developing a diabetic condition. In further embodiments of the invention, a level of glycated albumin greater than or equal to 9% of total albumin indicates a risk of developing a diabetic condition. In other embodiments, a level of glycated albumin greater than or equal to 13% of total albumin indicates a risk of developing a diabetic condition. Aspects of the invention also contemplate the assessment of log glycated albumin. In certain embodiments, a log percent glycated albumin greater than 2 indicates a risk of developing a diabetic condition. In still further embodiments, a log percent glycated albumin greater than 2.6 indicates a risk of developing diabetes.

The invention also encompasses additional assays and tests that further improve predictive accuracy when incorporated into the contemplated methods and panels. For example, certain embodiments of the invention comprise testing of blood glucose levels. Specifically, the level of glucose in the patient sample is compared to a threshold level of glucose. A level exceeding the predetermined threshold further indicates a risk of developing diabetes.

Any method known in the art is useful for the isolation and quantification of glucose. These include, but are not limited to, enzymatic assay, high-performance liquid chromatography (HPLC) and affinity chromatography, immunoassay, including quantification by radioassay,
enzyme-linked immunosorbent assay (ELISA), colorimetry, and electrochemical assays. Specific examples include enzyme-based test strips or electronic devices configured to quantify blood glucose levels. Further detail on such devices is provided in U.S. 2010/0204557. No matter the method used for quantification, the patient from whom the sample is drawn is usually deprived of food for a certain duration prior to taking the sample. For example, a patient may forego food for 8 hours prior to having blood drawn.

In some embodiments of the invention, a fasting glucose level greater than or equal to 70 mg/dl further indicates a risk of developing a diabetic condition. In further embodiments of the invention, a fasting glucose level greater than or equal to 80 mg/dl further indicates a risk of developing a diabetic condition. In other embodiments, a fasting glucose level greater than or equal to 90 mg/dl further indicates a risk of developing a diabetic condition. In still other embodiments, a fasting glucose level greater than 95 mg/dl further indicates a risk of developing a diabetic condition.

Embodiments of the invention may also incorporate the testing of adiponectin to further predict the future development of diabetes. Adiponectin is a protein hormone that modulates a number of metabolic processes, including glucose regulation and fatty acid oxidation. The hormone is typically secreted from adipose tissue into the bloodstream and compared to other hormones, is relatively abundant in plasma. In certain aspects of the invention, a level of adiponectin in the patient sample is compared to a threshold level of adiponectin. A level exceeding the predetermined threshold further indicates a risk of developing diabetes.

Any method known in the art is useful for the isolation and quantification of adiponectin. These include, but are not limited to, enzymatic assay, high-performance liquid chromatography (HPLC) and affinity chromatography, immunoassay, including quantification by radioassay, enzyme-linked immunosorbent assay (ELISA), colorimetry, and electrochemical assays. Further detail on such methods is provided in U.S. Patent Nos. 7,608,405 and 8,026,345.

In some embodiments of the invention, an adiponectin level greater than or equal to 2 log adiponectin further indicates a risk of developing a diabetic condition. In further embodiments of the invention, an adiponectin level greater than or equal to 2.1 log further indicates a risk of developing a diabetic condition. In other embodiments, an adiponectin level greater than or equal to 2.2 log further indicates a risk of developing a diabetic condition. In still other embodiments, an adiponectin level greater than 2.4 log further indicates a risk of developing a diabetic condition.

Other embodiments of the invention may also incorporate the testing of triglycerides to further predict the future development of diabetes. Triglycerides are a type of fat in the bloodstream and fat tissue. Triglycerides are formed from a single molecule of glycerol, combined with three molecules of fatty acid. In certain aspects of the invention, a level of adiponectin in the patient sample is compared to a threshold level of adiponectin. A level exceeding the predetermined threshold further indicates a risk of developing diabetes. Testing for triglycerides usually involves obtaining a blood sample from a patient who has fasted for a period of time.

Any method known in the art is useful for the isolation and quantification of triglycerides from the patient sample. These include, but are not limited to, enzymatic assay, high-performance liquid chromatography (HPLC) and affinity chromatography, immunoassay, including quantification by radioassay, enzyme-linked immunosorbent assay (ELISA), colorimetry, and electrochemical assays. Specific examples of assays suitable for the isolation and quantification of triglycerides are described in U.S. Patent Nos. 3,703,591; 4,245,041; and 4,999,289.

In some embodiments of the invention, a triglyceride level greater than or equal to 80 mg/dl further indicates a risk of developing a diabetic condition. In further embodiments of the invention, a triglyceride level greater than or equal to 90 mg/dl further indicates a risk of developing a diabetic condition. In other embodiments, a triglyceride level greater than or equal to 100 mg/dl further indicates a risk of developing a diabetic condition. In still other embodiments, a triglyceride level greater than or equal to 110 mg/dl further indicates a risk of developing a diabetic condition.

Certain aspects of the invention also encompass the use of information that is not necessarily obtained from a patient sample. The incorporation of this information into the encompassed methods and panels can further improve predictive accuracy. In certain embodiments, the body mass index (BMI) of the patient is determined. The BMI of the patient is compared to a threshold level BMI. A level exceeding the predetermined threshold further indicates a risk of developing diabetes. The BMI test assesses the weight of a patient relative to his or her height and based on the ratio, classifies the patient as underweight, normal weight, overweight, or obese. The calculation of BMI is well known in the art. To obtain results from the BMI test, a patient's weight in kilograms (kg) is divided by his height in meters (m), and then again divided by his height in meters (m).

In some embodiments of the invention, a BMI greater than or equal to 10 kg/m$^2$ further indicates a risk of developing a diabetic condition. In further embodiments of the invention, a BMI greater than or equal to 20 kg/m$^2$ further indicates a risk of developing a diabetic condition. In other embodiments, a BMI greater than or equal to 27 kg/m$^2$ further indicates a risk of developing a diabetic condition.

Additional parameters include whether or not the patient has a family history of diabetes. A family history of diabetes further indicates a risk of developing a diabetic condition at a future time. Information regarding the patient's family history can be obtained by simply asking the patient for such information, either verbally or through a printed questionnaire, for example.

Certain embodiments of the invention also incorporate determining whether or not the patient has received statin therapy. Statins are a class of drugs used to lower cholesterol levels by inhibiting certain enzymes. While statins help treat cardiovascular disease, statin therapy may also contribute to diabetes. As encompassed by the invention, the existence of statin therapy further indicates a risk of developing a diabetic condition. Information regarding prior statin therapy can be obtained by simply asking the patient for such information, either verbally or through a printed questionnaire, for example.

In certain aspects, a patient's risk of developing diabetes is determined via the analysis of the aforementioned parameters performed in accordance with a diabetes predictive algorithm of the present invention. The contemplated algorithm has been developed to assist the medical practitioner, as well as the patient, in the management of the patient's healthcare. The algorithm addresses certain factors or parameters in order to make a comprehensive evaluation and facilitates an accurate prediction based on statistical data accumulated from a broad range of studies. Utilization of such an algorithm to perform an analysis for diabetes prediction not only provides a more accurate assessment, but also expedites the process faster than conventional analysis. This savings in time would not only lead to earlier treatment of the patient, but also would provide potential cost savings (e.g., to the patient, the insurance companies, the medical facilities, and/or the healthcare practitioner). Further, such use of an algorithm could lead to standardization of the practice and reduce the likelihood of misdiagnosis.

The predictive risk analysis may comprise a series of iterative steps, with each successive step evaluating each new data (i.e., result of a test from the extended risk panel) in combination with all data submitted in the previous steps of the cycle and relevant information about the patient initially submitted (said evaluation being performed in comparison to statistical data included as part of the algorithm to facilitate this process), until all test data entered or submitted for analysis are evaluated comprehensively. Upon completion of the final step, the analysis function terminates, and the predictive result is formed upon completion of the analysis function. The present invention also contemplates the modification or update of statistical data, which is included as part of the algorithm, as such data becomes available and would serve to improve the accuracy of prediction.

The predictive algorithm of the present invention may be embodied in any suitable application, such as a computer program or code that can facilitate its use. The algorithm or application embodying the application may be stored in the internal or external hard drive of a computer, a portable drive or disc, a server, a temporary or permanent memory device, or any other storage means that can facilitate the use of the algorithm and/or the results derived from its use. The algorithm or application is preferably in communication with at least one processing device that facilitates the predictive analysis, for example, a computer or network processor. The algorithm or associated application may be accessed locally (e.g., on a single or networked computer) or remotely (e.g., web-based network via the internet, or via the intranet).

This access to the algorithm or application may be facilitated via the use of any suitable equipment, including without limitation, a computer, an internet appliance, telephonic device, a wireless device, and the like. Access to the algorithm, the application embodying the algorithm or the results obtained from use of the algorithm may be secured or limited from general access or use via a password, encryption, biometric- or voice-activation, or other suitable means of protection.

A patient's personal information, including, but not limited to , name, address, age, contact information, prior medical history, and/or clinical data may be entered or submitted, locally or remotely for processing, which includes performance of the predictive analysis. The results from the processing step may be obtained by, or delivered and transmitted to, an authorized party, such as a healthcare professional, a healthcare facility or its employees, the patient or the patient's guardian, or the patient's insurance company.

The obtaining of delivery of results may be performed locally or remotely, and the results may be in digital, print, or any other suitable format, and may be protected or secured via any suitable means. The delivery or transmission of results may be automated, for example, with respect to delivery or transmission time and to the relevant authorized parties. The results may be delivered or transmitted using any suitable means, including without limitation, the Internet, an intranet, an electronic health record or management interface, telephone (land line, wireless, or VOIP, email, facsimile, postal mail, or in person. Patient results may also be coded to protect confidentiality. Such coding may be conducted in addition to any aforementioned security or protection measures.

As contemplated by the invention, the functions and embodiments described above can be implemented using software, hardware, firmware, hard wiring, or any combinations of these. Features implementing functions can also be physically located at various positions, including being distributed such that portions of functions are implemented at different physical locations.

As one skilled in the art would recognize as necessary or best-suited for performance of the methods of the invention, a computer system or machines include one or more processors (e.g., a central processing unit (CPU) a graphics processing unit (GPU) or both), a main memory and a static memory, which communicate with each other via a bus.

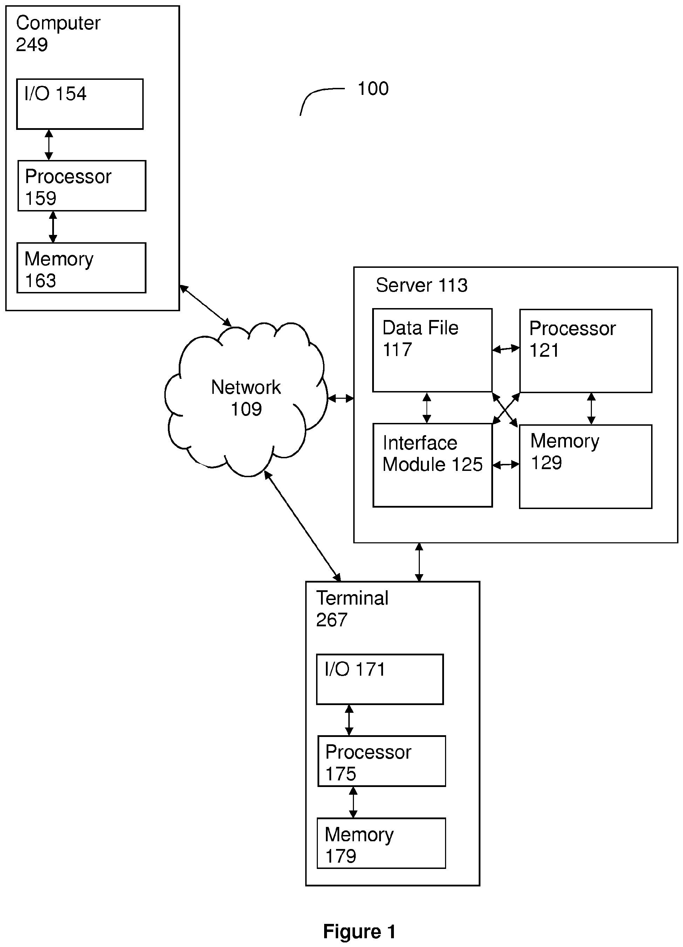

In an exemplary embodiment shown in FIG. 1, system **100** can include a computer **149** (e.g., laptop, desktop, tablet, or smartphone). The computer **149** may be configured to communicate across a network **109.** Computer **149** includes one or more processor **159** and memory **163** as well as an input/output mechanism **154.** Where methods of the invention employ a client/server architecture, steps of methods of the invention may be performed using server **113,** which includes one or more of processor **121** and memory **129,** capable of obtaining data, instructions, etc., or providing results via interface module **125** or providing results as a file **117.** Server **113** may be engaged over network **109** through computer **149** or terminal **167,** or server **113** may be directly connected to terminal **167,** including one or more processor **175** and memory **179,** as well as input/output mechanism **171.**

System **100** or machines may further include, for any of I/O **149, 137,** or **171** a video display unit (e.g., a liquid crystal display (LCD) or a cathode ray tube (CRT)). Computer systems or machines can also include an alphanumeric input device (e.g., a keyboard), a cursor control device (e.g., a mouse), a disk drive unit, a signal generation device (e.g., a speaker), a touchscreen, an accelerometer, a microphone, a cellular radio frequency antenna, and a network interface device, which can be, for example, a network interface card (NIC), Wi-Fi card, or cellular modem.

Memory **163, 179,** or **129** can include a machine-readable medium on which is stored one or more sets of instructions (e.g., software) embodying anyone or more of the methodologies or functions described herein. The software may also reside, completely or at least partially, within the main memory and/or within the processor during execution thereof by the computer system, the main memory and the processor also constituting machine-readable media. The software may further be transmitted or received over a network via the network interface device.

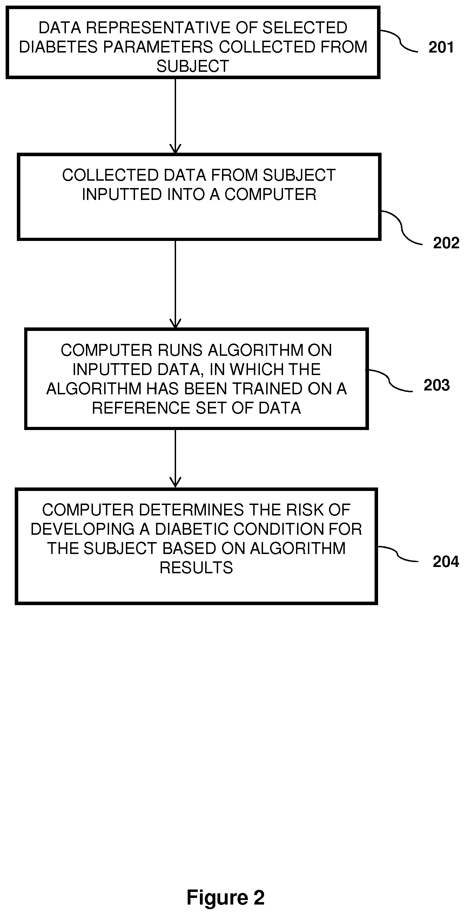

Exemplary step-by-step methods are described schematically in Figure 2. It will be understood that of the methods described herein, as well as any portion of the systems and methods disclosed herein, can be implemented by computer, including the devices described above. Information is collected from the patient regarding the selected diabetes-associated parameters or biomarkers **201.** This data is then inputted into the central processing unit (CPU) of a computer **202.** The CPU is coupled to a storage or memory for storing instructions for implementing methods of the present invention, such as the diabetes predictive algorithm. The instructions, when executed by the CPU, cause the CPU to predict the patient's risk of developing a diabetic condition at a future time. The CPU provides this determination by inputting the subject data into an algorithm trained on a reference set of data from a plurality of subjects for whom diabetes-associated parameters/biomarkers and disease outcomes are known 203. The reference set of data may be stored locally within the computer, such as within the computer memory. Alternatively, the reference set may be stored in a location that is remote from the computer, such as a server. In this instance, the computer communicates across a network to access the reference set of data. The CPU then predicts the patient's risk of developing diabetes at a later point in time based on the data entered into the algorithm.

After selecting the appropriate parameters or biomarkers as described herein, well-known techniques such as cross-correlation, Principal Components Analysis (PCA), factor rotation, Logistic Regression (LogReg), Linear Discriminant Analysis (LDA), Eigengene Linear Discriminant Analysis (ELDA), Support Vector Machines (SVM), Random Forest (RF), Recursive Partitioning Tree (RPART), related decision tree classification techniques, Shrunken Centroids (SC), StepAIC, Kth-Nearest Neighbor, Boosting, Decision Trees, Neural Networks, Bayesian Networks, Support Vector Machines, and Hidden Markov Models, Linear Regression or classification algorithms, Nonlinear Regression or classification algorithms, analysis of variants (ANOVA), hierarchical analysis or clustering algorithms; hierarchical algorithms using decision trees; kernel based machine algorithms such as kernel partial least squares algorithms, kernel matching pursuit algorithms, kernel Fisher's discriminate analysis algorithms, or kernel principal components analysis algorithms, or other mathematical and statistical methods can be used to develop the predictive algorithm. A selected population of individuals (i.e., a reference set or reference population) is used to train the algorithm, where historical information is available regarding the values of the selected parameters in the population and their clinical outcomes. To calculate a risk of developing a diabetic condition for a given individual, parameter values are obtained from one or more samples collected from the individual and/or from non-biological sources (i.e. completed questionnaires, etc.) obtained from the individual and used as input data (inputs into a predictive algorithm fitted to the actual historical data obtained from the selected population of individuals).

Any formula or algorithm may be used to combine selected parameter results into indices useful in the practice of the invention. As indicated above, and without limitation, such indices may indicate, among the various other indications, the probability, likelihood, absolute or relative risk, time to or rate of conversion from one disease state to another.

Although various preferred formula are described here, several other model and formula types beyond those mentioned herein and in the definitions above are well known to one skilled in the art. The actual model type or formula used may itself be selected from the field of potential models based on the performance and diagnostic accuracy characteristics of its results in a training population. The specifics of the formula itself may commonly be derived from selected parameter results in the relevant training population. Amongst other uses, such formula may be intended to map the feature space derived from one or more selected parameter inputs to a set of subject classes (e.g. useful in predicting class membership of subjects as normal, pre-Diabetes, Diabetes), to derive an estimation of a probability function of risk using a Bayesian approach (e.g. the risk of Diabetes), or to estimate the class-conditional probabilities, then use Bayes' rule to produce the class probability function as in the previous case.

Preferred formulas include the broad class of statistical classification algorithms, and in particular the use of discriminant analysis. The goal of discriminant analysis is to predict class membership from a previously identified set of features. In the case of linear discriminant analysis (LDA), the linear combination of features is identified that maximizes the separation among groups by some criteria. Features can be identified for LDA using an eigengene based approach with different thresholds (ELDA) or a stepping algorithm based on a multivariate analysis of variance (MANOVA). Forward, backward, and stepwise algorithms can be performed that minimize the probability of no separation based on the Hotelling-Lawley statistic.

Eigengene-based Linear Discriminant Analysis (ELDA) is a feature selection technique developed by Shen et al. (2006). The formula selects features (e.g. parameters) in a multivariate framework using a modified eigen analysis to identify features associated with the most important eigenvectors. "Important" is defined as those eigenvectors that explain the most variance in the differences among samples that are trying to be classified relative to some threshold.

A support vector machine (SVM) is a classification formula that attempts to find a hyperplane that separates two classes. This hyperplane contains support vectors, data points that are exactly the margin distance away from the hyperplane. In the likely event that no separating hyperplane exists in the current dimensions of the data, the dimensionality is expanded greatly by projecting the data into larger dimensions by taking non-linear functions of the original variables (Venables and Ripley, 2002). Although not required, filtering of features for SVM often improves prediction. Features (e.g., parameters/biomarkers) can be identified for a support vector machine using a non-parametric Kruskal-Wallis (KW) test to select the best univariate features. A random forest (RF, Breiman, 2001) or recursive partitioning (RPART, Breiman et al., 1984) can also be used separately or in combination to identify biomarker combinations that are most important. Both KW and RF require that a number of features be selected from the total. RPART creates a single classification tree using a subset of available biomarkers.

Other formula may be used in order to pre-process the results of individual selected parameter measurement into more valuable forms of information, prior to their presentation to the predictive formula. Most notably, normalization of parameter results, using either common mathematical transformations such as logarithmic or logistic functions, as normal or other distribution positions, in reference to a population's mean values, etc. are all well known to those skilled in the art. Of particular interest are a set of normalizations based on parameters not derived from biological samples such as age, gender, race, or sex, where specific formula are used solely on subjects within a class or continuously combining such a parameter as an input. In other cases, sample based parameters can be combined into calculated variables (much as BMI is a calculation using Height and Weight) which are subsequently presented to a formula.

In addition to the individual parameter values of one subject potentially being normalized, an overall predictive formula for all subjects, or any known class of subjects, may itself be recalibrated or otherwise adjusted based on adjustment for a population's expected prevalence and mean parameter values, according to the technique outlined in D'Agostino et al. (2001) JAMA 286:180-187, or other similar normalization and recalibration techniques. Such epidemiological adjustment statistics may be captured, confirmed, improved and updated continuously through a registry of past data presented to the model, which may be machine readable or otherwise, or occasionally through the retrospective query of stored samples or reference to historical studies of such parameters and statistics. Additional examples that may be the subject of formula recalibration or other adjustments include statistics used in studies by Pepe, M. S. et al, 2004 on the limitations of odds ratios; Cook, N. R., 2007 relating to ROC curves; and Vasan, R. S., 2006 regarding biomarkers of cardiovascular disease. In addition, , the numeric result of a classifier formula itself may be transformed post-processing by its reference to an actual clinical population and study results and observed endpoints, in order to calibrate to absolute risk and provide confidence intervals for varying numeric results of the classifier or risk formula.

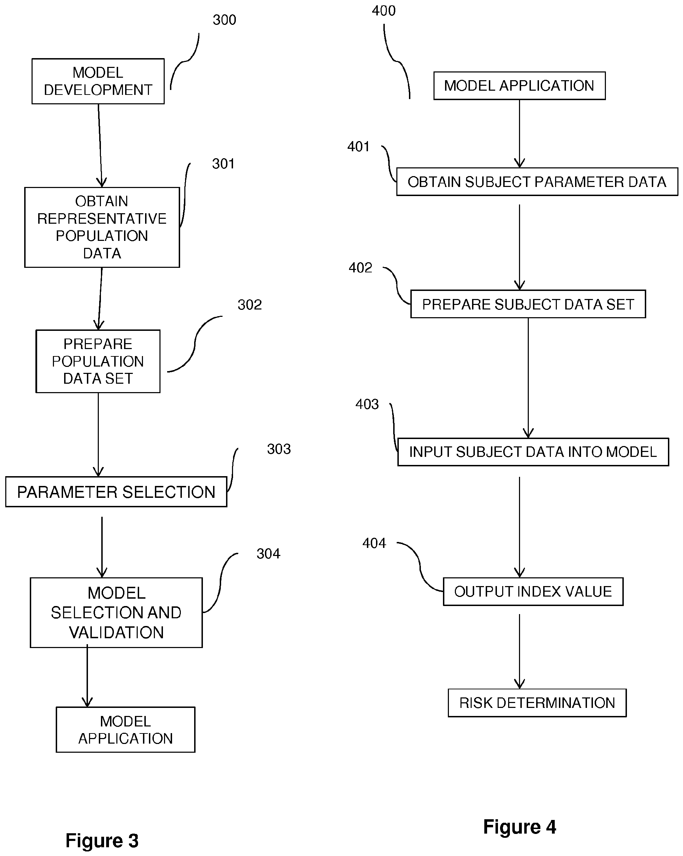

FIG. 3 depicts a flow diagram representing an exemplary method 300 for developing a model which may be used to evaluate a risk of a person, or group of people, for developing a diabetic condition. The method 300 may be implemented using the example computing system environment 100 of FIG. 1 and will be used to explain the operation of the environment 100. However, it should be recognized that the method 300 could be implemented by a system different than the computing system environment 100. At a block 301, parameter data from a representative population, as has been described herein, is obtained from a data storage device, such as the system memory 129, an internal or external database, or other computer storage media. The parameter or biomarker data may be initially derived through a variety of means, including prospective (longitudinal) studies that involve observing a representative population over a period of time, retrospective studies of a representative population that queries population samples and/or a retrospective epidemiological database containing the study results (e.g. an NIH database). The parameter data may be derived from a single study or multiple studies, and generally includes data pertaining to the desired indication and endpoint of the representative population, including values of the parameters described herein, clinical annotations (which may include endpoints), and most particularly the desired endpoints for training an algorithm for use in the invention, across many subjects.

At a block 302, the representative population data set is prepared as needed to meet the requirements of the model or analysis that will be used for parameter selection, as described below. For example, data set preparation may include preparing the parameter values from each subject within the representative population, or a chosen subset thereof. When necessary, various data preparation methods may be used to prepare the data prior to training the model, such as gap fill techniques (e.g., nearest neighbor interpolation or other pattern recognition), quality checks, data combination using of various formulae (e.g., statistical classification algorithms), normalization and/or transformations, such as logarithmic functions to change the distribution of data to meet model requirements (e.g., base 10, natural log, etc.). Again, the particular data preparation procedures are dependent upon the model or models that will be trained using the representative population data. The particular data preparation techniques for various different model types are known, and need not be described further.

At a block 303, the particular parameters are selected to be subsequently used in the training of the model used to evaluate a risk of developing a diabetic condition. Parameter selection may involve utilizing a selection model to validate the representative population data set and selecting the parameter data from the data set that provides the most reproducible results. Examples of data set validation may include, but are not limited to, cross-validation and bootstrapping. From the parameter selection, the model to be used in evaluating a risk of developing a diabetic condition may be determined and selected. However, it is noted that not all models provide the same results with the same data set. For example, different models may utilize different numbers of parameters and produce different results, thereby adding significance to the combination of biomarkers on the selected model. Accordingly, multiple selection models may be chosen and utilized with the representative population data set, or subsets of the data set, in order to identify the optimal model for risk evaluation. Examples of the particular models, including statistical models, algorithms, etc., which may be used for selecting the parameters have been described above.

For each selection model used with the data set, or subset thereof, the parameters are selected based on each parameter's statistical significance in the model. When inputted into each model, the parameters are selected based on various criteria for statistical significance, and may further involve cumulative voting and weighting. Tests for statistical significance may include exit-tests and analysis of variance (ANOVA). The model may include classification models (e.g., LDA, logistic regression, SVM, RF, tree models, etc.) and survival models (e.g., cox), many examples of which have been described above.

It is noted that while parameters may be applied individually to each selection model to identify the statistically significant parameters, in some instances individual parameters alone may not be fully indicative of a risk for a diabetic condition, in which case combinations of parameters may be applied to the selection model. For example, rather than utilizing univariate parameter selection, multivariate parameter selection may be utilized. That is, a parameter may not be a good indicator when used as a univariate input to the selection model, but may be a good indicator when used in combination with other parameter (i.e., a multivariate input to the model), because each parameter may bring additional information to the combination that would not be indicative if taken alone.

At a block 304, the model to be used for evaluating risk is selected, trained and validated. In particular, leading candidate models may be selected based on one or more performance criteria, examples of which have been described above. For example, from using the data set, or data subsets, with various models, not only are the models used to determine statistically significant parameters, but the results may be used to select the optimal models along with the parameters. As such, the evaluation model used to evaluate risk may include one of those used as a selection model, including classification models and survival models. Combinations of models markers, including marker subsets, may be compared and validated in subsets and individual data sets. The comparison and validation may be repeated many times to train and validate the model and to choose an appropriate model, which is then used as an evaluation model for evaluating risk of a diabetic condition.

FIG. 4 is a flow diagram of an example method 400 for using a model to evaluate a risk of a subject (e.g., a person, or group of people) developing a diabetic condition. At a block 401, parameter data from the subject is obtained from a data storage device, which may be the same as, or different from, the data storage device discussed above with reference to FIG. 3. The subject parameter data may be initially derived through a variety of means, including self-reports questionnaires, physical examination, laboratory testing and existing medical records, charts, databases, and/or patient samples. As with the representative population parameter data at block 302 of FIG. 3, the subject parameter data at block 402 may be prepared using transforms, logs, combinations, normalization, etc. as needed according to the model type selected and trained in FIG. 34. Once the data has been prepared, at a block **403,** the subject biomarker data is input into the evaluation model, and at a block **404** the evaluation model outputs an index value (e.g., risk score, relative risk, time to conversion, etc.).

Enzymatic assays, high-performance liquid chromatography (HPLC) and affinity chromatography, immunoassays, including quantification by radioassay, enzyme-linked immunosorbent assays (ELISA), colorimetry, and electrochemical assays may be used to detect levels of biomarkers (e.g. glycated albumin; glucose; adiponectin; triglyceride). These assays are described in more detail below.

Enzymatic assays are assays for measuring enzymatic activity over a period of time. The activity of an enzyme may be measured by determine the rate of product formation or substrate depletion during an enzyme-catalyzed reaction. There are several different enzymatic assay procedures that can be utilized to measure and detect a level of a biomarker in a sample. The following four experiments are typically used: initial rate experiments, progress curve experiments, transient kinetic experiments and relaxation experiments. Initial rate experiments involve mixing an enzyme with a large excess of substrate such that the enzyme-substrate intermediate builds up in a fast initial transient. Then the reaction achieves a steady-state kinetics in which enzyme substrate intermediates remains approximately constant over time and the reaction rate changes relatively slowly. Rates are measured for a short period after the attainment of the quasi-steady state, typically by monitoring the accumulation of product with time. Because the measurements are carried out for a very short period and because of the large excess of substrate, the approximation that the amount of free substrate is approximately equal to the amount of the initial substrate can be made. The initial rate experiment is the simplest to perform and analyze, being relatively free from complications such as back-reaction and enzyme degradation. In progress curve experiments, the kinetic parameters are determined from expressions for the species concentrations as a function of time. The concentration of the substrate or product is recorded in time after the initial fast transient and for a sufficiently long period to allow the reaction to approach equilibrium. In transient kinetics experiments, reaction behavior is tracked during the initial fast transient as the intermediate reaches the steady-state kinetics period. These experiments are more difficult to perform than either of the above two classes because they require specialist techniques (such as flash photolysis of caged compounds) or rapid mixing (such as stopped-flow, quenched flow or continuous flow). In relaxation experiments, an equilibrium mixture of enzyme, substrate and product is perturbed, for instance by a temperature, pressure or pH jump, and the return to equilibrium is monitored. The analysis of these experiments requires consideration of the fully reversible reaction. Moreover, relaxation experiments are relatively insensitive to mechanistic details and are thus not typically used for mechanism identification, although they can be under appropriate conditions.

High-performance liquid chromatography (HPLC) may also be used to detect biomarkers. HPLC relies on pumps to pass a pressurized liquid and a sample mixture through a column filled with a sorbent, leading to the separation of the sample components. The active component of the column, the sorbent, is typically a granular material made of solid particles (e.g. silica, polymers, etc.), 2-50 micrometers in size. The components of the sample mixture are separated from each other due to their different degrees of interaction with the sorbent particles. The pressurized liquid is typically a mixture of solvents (e.g. water, acetonitrile and/or methanol) and is referred to as "mobile phase". Its composition and temperature plays a major role in the separation process by influencing the interactions taking place between sample components and sorbent. These interactions are physical in nature, such as hydrophobic (dispersive), dipole-dipole and ionic, most often a combination thereof. The schematic of an HPLC instrument typically includes a sampler, pumps, and a detector. The sampler brings the sample mixture into the mobile phase stream which carries it into the column. The pumps deliver the desired flow and composition of the mobile phase through the column. The detector generates a signal proportional to the amount of sample component emerging from the column, hence allowing for quantitative analysis of the sample components. A digital microprocessor and user software control the HPLC instrument and provide data analysis. Some models of mechanical pumps in a HPLC instrument can mix multiple solvents together in ratios changing in time, generating a composition gradient in the mobile phase. Various detectors are in common use, such as UV/Vis, photodiode array (PDA) or based on mass spectrometry. Most HPLC instruments also have a column oven that allows for adjusting the temperature the separation is performed at.

Another technique for detecting biomarkers of the invention includes using affinity chromatography. Affinity chromatography is a known technique used to isolate molecules from a complex mixture using highly-specific biological interactions between two molecules. The interactions include those between enzyme and substrate, receptor and ligand, or antibody and antigen. Typically, one of the molecules is attached to a substrate, and is used to capture a target molecule from the sample. The substrate is typically an affinity column. Successful affinity purification requires a certain degree of knowledge and understanding of the nature of interactions between the target molecule and the ligand to help determine the selection of an appropriate affinity ligand and purification procedure. In certain embodiments, a sample is introduced to a substrate containing a capture molecule specific to a target molecule (e.g. biomarkers of the invention). While the sample interacts with the substrate, the target molecules attached to the capture molecule, while other sample contents do not bind. Unbound sample is removed, leaving the target molecules isolated. The target molecules may then be detected using methods known in the art (probe-based detection (i.e. fluorescence intensity), sequencing, colorimetry, etc).

Immunoassays, similar to affinity chromatography, rely on the ability of a capture molecule, such as an antibody, to recognize and couple to a target molecule within a sample. In other words, the capture molecule forms a complex with the target molecule within the sample, and the complex can be detected in order to detect the target molecule. Usually, the formed complexes are separated from unbound components in the sample prior to detection. Typically, labels are attached to the antibodies or antigens that allow for detection of the target molecule via the label. Immunoassays that employ enzymes as labels are referred to as enzyme-linked immunosorbent assays. Certain immunoassays may incorporate real-time quantitative polymerase chain reaction (RTqPCR), and use nucleic acid probes that are detectable when subject to PCR. Radioactive isotopes can be incorporated into immunoassay reagents to produce a radioimmunoassay (RIA). Radioactivity emitted by bound antibody-antigen complexes can be easily detected using conventional methods, thereby allowing detection of a target molecule.

Colorimetry, also referred to as colorimetric analysis, is a method of determining the concentration of an element, such as a target molecule or molecule attached to the target molecule, with the aid of a color reagent. The element and the reagent form a complex that emits a color, and the concentration of the element can be determined based on the intensity of the color.

Examples

Example 1: Prior Art Diabetes Predictive Panels

Risk prediction for various diabetic conditions has also encompass multi-variate risk prediction algorithms and computed indices that assess and estimate a subject's risk for developing such conditions with reference to a historical cohort. Risk assessment using such predictive mathematical algorithms and computed indices has increasingly been incorporated into guidelines for diagnostic testing and treatment, and encompass indices obtained from and validated with, inter alia, multi-stage, stratified samples from a representative population. A number of conventional diabetes risk factors or parameters have been incorporated into these predictive models. Notable examples of such algorithms include the San Antonio Heart Study (Stern, M. P. et al, (1984) Am. J. Epidemiol. 120: 834-851; Stern, M. P. et al, (1993) Diabetes 42: 706-714; Burke, J. P. et al, (1999) Arch. Intern. Med. 159: 1450-1456), the Framingham study (Wilson et al., Arch Intern Med. 2007 may 28; 167(10): 1068-74), the Atherosclerosis Risk in Communities (ARIC) Study (Schmidt et al., Diabetes Care 2005 Aug; 28(8): 2013-8), the Multi-Ethnic Study of Atherosclerosis (MESA)(Mann et al., Am J Epidemiol. 2010 May 1; 171 (9): 980-8), and the combined Inter99/Botnia Diabetes Study (Kolberg et al., Diabetes Care. 2009 Jul; 32(7): 1207-12), the contents of each are expressly incorporated herein by reference. The results of these studies, including the final selected parameters, equations, and predictive accuracies as measured by C statistic, is provided below in Table 1. None of these studies assessed a period greater than eight years.

**Table 1: Listing of Prior Predictive Equations**

**In all equations the probability of developing diabetes = $1/\exp^{\log X+1}$**

**A) San Antonio Heart Study Logistic Regression Study** (uses 1) age, 2) gender, 3) ethnic background if Mexican, 4) fasting glucose, 5) systolic blood pressure, 6) high density lipoprotein cholesterol (HDL-C), 7) body mass index, and 8) family history of diabetes) (reference 7). The best C statistic with these parameters was 0.843.
X = -13.415 + 0.028 X age in years + 0.661 X sex (1 if female, else 0) + 0.412 X (1 if Mexican, else 0) + 0.079 X fasting glucose in mg/dL + 0.018 X systolic blood pressure in mmHg - 0.039 X HDL-C in mg/dL + 0.076 X body mass index in kg/m2 + 0.481 X family history of diabetes (else 0).

**B) Framingham Offspring Study Logistic Regression Study** (uses 1) fasting glucose status, 2) body mass index as a measure of being overweight or obesity, 3) HDL-C, 4) triglycerides, 5) blood pressure, and 6) family history of diabetes) (reference 12). The best C statistic with these parameters was 0.850.
X = -5.517 + 1.98 X (1 if impaired glucose, else 0) + 0.30 X (1 if overweight, else 0) + 0.92 X (1 if obese, else 0) + 0.94 X (1 if HDL-C decreased, else 0) + 0.58 X (1 if triglycerides increased, else 0) + 0.50 X (1 if blood pressure is elevated, else is 0) + 0.57 X (1 if family history of diabetes, else 0).

**C) Atherosclerosis Risk in Communities (ARIC) Study Logistic Regression Study** (uses 1) age, 2) ethnic background if Afro-American, 3) family history of diabetes, 4) fasting glucose, systolic blood pressure, 5) waist circumference, 6) height, 7) HDL-C, and 8) triglycerides). The best C statistic was 0.80.

X = -9.9808 + 0.0173 X age in years + 0.4433 X if black + 0.4981 X 1 if family history of diabetes + 0.0880 X fasting glucose in mg/dL + 0.0111 X systolic blood pressure in mmHg + 0.0273 X waist circumference in cm - 0.0326 X height in cm - 0.0122 X HDL-C in mg/dL + 0.00271 X triglycerides in mg/dL.

**D) Best Fit MESA/ARIC Logistic Regression Model** (uses same parameters as ARIC Study). The best C statistic was 0.84.

X = -12.911 + 0.305 X age in years + 0.181 X 1 if black + 0.578 X 1 if family history of diabetes + 0.119 X fasting glucose in mg/dL + 0.006 X systolic blood pressure in mmHg + 0.028 X waist in cm - 0.009 X HDL-C in mg/dL + 0.001 X triglycerides in mg/dL.

**E) Inter99 Diabetes Prediction Score** (uses the following biochemical parameters: 1) 1) glycosylated hemoglobin, 2) fasting glucose, 3) adiponectin, 4) C reactive protein, 5) insulin, 6) ferritin, 7) interleukin 2 receptor alpha. No equation was provided. The best C statistic was 0.84 in the Inter99 study. In the followup Botnia study, the C statistic was 0.78.

Despite the numerous studies and algorithms that have been used to assess the risk of various diabetic conditions, the need for more accurate panels and methods for assessing such risks or conditions still remains. As shown in Table 1, for example, none of the listed prior art methods reported a C statistic greater than 0.85.

Example 2: Reference Set

A reference set of 2,620 men and women with a mean age of 58 years were followed for 8.5 years. All subjects at the commencement of the study did not have diabetes, defined in this study as a fasting serum glucose value of > 125 mg/dl and/or receiving treatment for diabetes. From this population, 186 subjects (7.1%) developed diabetes at some point within the 8.5 year period. A number of parameters were evaluated among the converters (those who developed diabetes within the study period) and non-converters (those who did not develop diabetes within the study period), including age, body mass index, waist, glycated albumin, log glycated albumin, adiponectin, log adiponectin, C-reactive protein, log C-reactive protein, HDL-cholesterol, triglycerides, log triglycerides, parental diabetes, fasting glucose, female, hypertensive, insulin, log insulin, and uric acid treatment. The Oral Glucose Tolerance Test was not among the initially selected parameters. A breakdown of the converters and non-converters according to the assessed parameters is provided in Table 2 below.

**Table 2. Study Subjects (n=2,620)**

| Subject Variables* | Non-Converters (n=2,234) | Converters (n=186) |
|---|---|---|
| Age (years) | 57.63 (9.63) | 59.46 (8.64)[c] |
| Body Mass Index (kg/m$^2$) | 27.27 (4.71) | 31.38 (5.41)[a] |
| Waist (cm) | 95.76 (12.83) | 107.12 (12.06)[a] |
| Glycated Albumin (%)& | 14.0 [13.2,15.0] | 14.4 [13.6,15.3][b] |
| Log Glycated Albumin (%) | 2.64 (0.1) | 2.68 (0.11)[a] |
| Adiponectin (ug/ml)& | 11.9 [8.4,17.1] | 8.2 [6.2,11.0][a] |
| Log Adiponectin (ug/ml) | 2.49 (0.51) | 2.13 (0.44)[a] |
| C Reactive Protein (mg/L)& | 1.84 [0.84,17.1] | 3.27 [1.55,6.88][b] |
| Log C Reactive Protein (mg/L) | 0.66 (1.14) | 1.16 (1.12)[a] |
| HDL-cholesterol (mg/dL) | 52.69 (15.77) | 43.55 (11.98)[a] |
| Triglycerides (mg/dL)& | 110.0 [78.0,155.0] | 155.0 [110.0,207.0][a] |
| Log Triglycerides (mg/dL) | 4.7 (0.49) | 5.03 (0.50)[a] |
| Parental Diabetes (%) | 22.36% | 38.26%[d] |
| Fasting Glucose (mg/dL) | 95.79 (9.12) | 110.64 (9.09)[b] |
| Female (%) | 55.22% | 41.94%[b] |
| Hypertensive (%) | 35.56% | 54.84%[a] |
| Insulin (microU/ml) | 10.2 [8.0, 13.4] | 15.3 [11.2,20.4][a] |
| Log Insulin | 2.36 (0.4) | 2.71 (0.42)[a] |

(continued)

| Subject Variables* | Non-Converters (n=2,234) | Converters (n=186) |
|---|---|---|
| Uric Acid Treatment (%) | 1.27% | 1.61% |

*Mean Values (standard deviation) &
Median (interquartile ranges)
[a] p<0.0001, [b] p<0.001, [c] p<0.05

Example 3: Algorithm Construction

The parameters selected above were then evaluated using stepwise regression analysis. SAS LOGISTIC Procedure was used to perform the analysis as shown below:

The LOGISTIC Procedure

Model Information

| Data Set | ES_DIAB.DIABETES_MASTER_V1 | |
|---|---|---|
| Response Variable | incD | IncDiab |
| Number of Response Levels | 2 | |
| Model | binary logit | |
| Optimization Technique | Fisher's scoring | |
| Number of Observations Read | | 2839 |
| Number of Observations Used | | 1971 |

Response Profile

| Ordered Value | incD | Total Frequency |
|---|---|---|
| 1 | 1 | 148 |
| 2 | 0 | 1823 |

Probability modeled is incD=1.
NOTE: 868 observations were deleted due to missing values for the response or explanatory variables.

Model Convergence Status

Convergence criterion (GCONV=1E-8) satisfied.

Model Fit Statistics

| Criterion | Intercept Only | Intercept and Covariates |
|---|---|---|
| AIC | 1052.967 | 653.683 |
| SC | 1058.553 | 698.373 |
| -2 Log L | 1050.967 | 637.683 |

Testing Global Null Hypothesis: BETA=0

| Test | Chi-Square | DF | Pr > ChiSq |
|---|---|---|---|
| Likelihood Ratio | 413.2836 | 7 | <.0001 |
| Score | 415.8924 | 7 | <.0001 |
| Wald | 228.8724 | 7 | <.0001 |

Analysis of Maximum Likelihood Estimates

| Parameter | DF | Standard Estimate | Error | Wald Chi-Square | Pr > ChiSq |
|---|---|---|---|---|---|
| Intercept | 1 | -30.0077 | 3.0639 | 95.9203 | <.0001 |
| GLUC6 | 1 | 0.1543 | 0.0126 | 149.4746 | <.0001 |
| BMI6 | 1 | 0.0905 | 0.0205 | 19.4197 | <.0001 |
| TG6 | 1 | 0.00365 | 0.00129 | 7.9868 | 0.0047 |
| parent_diab | 1 | 0.6940 | 0.2225 | 9.7276 | 0.0018 |
| logga_perc | 1 | 3.7138 | 1.0035 | 13.6952 | 0.0002 |
| logadipo | 1 | -0.7934 | 0.2424 | 10.7102 | 0.0011 |
| CHOLRX6 | 1 | 0.4669 | 0.2741 | 2.9009 | 0.0885 |

Odds Ratio Estimates

| Effect | Point Estimate | 95% Wald Confidence | Limits |
|---|---|---|---|
| GLUC6 | 1.167 | 1.138 | 1.196 |
| BMI6 | 1.095 | 1.052 | 1.140 |
| TG6 | 1.004 | 1.001 | 1.006 |
| parent_diab | 2.002 | 1.294 | 3.096 |
| logga_perc | 41.009 | 5.737 | 293.156 |
| logadipo | 0.452 | 0.281 | 0.727 |
| CHOLRX6 | 1.595 | 0.932 | 2.729 |

Association of Predicted Probabilities and Observed Responses

| Percent Concordant | 91.3 | Somers' D | 0.831 |
|---|---|---|---|
| Percent Discordant | 8.2 | Gamma | 0.835 |
| Percent Tied | 0.5 | Tau-a | 0.116 |
| Pairs | 269804 | c | 0.916 |

After the analysis was conducted, only seven parameters remained statistically significant:

1) Glucose (GLUC6 above): For every 1 mg/dl increase of glucose > 95.79, the odds ratio increases 1.174 and decreases by the same amount for every 1 mg/dl < 95.79. Therefore, for every 10 mg/dl fasting glucose increase > 95.79, the risk of developing diabetes over a 10 year period increases 11.7 fold.
2) Body Mass Index (BMI6 above): For every 1 unit increase > 27.27 kg/m$^2$, the odds ratio increases by 1.079 and decreases by the same amount for every 1 unit decrease < 27.27 kg/m$^2$. Therefore, for a 5 unit increase up to 32.27 kg/m$^2$ in BMI > 27.27 kg/m$^2$ risk of diabetes over a 10 year period increases 5.4 fold.

3) Log Percent Glycated Albumin (logga_perc above): For every 1 unit increase > 2.64, the odds ratio increases 41.01 and decreases by the same amount for every one unit < 2.64. This translates into a 41 fold increased risk of diabetes for a major increase in glycated albumin > 13.5%.

4) Log Adiponectin (logadipo above): For every 1 unit increase > 2.49, the odds ratio decreases 0.452 and increases by the same amount for every 1 unit decrease < 2.49.

5) Parental History of Diabetes (parent_diab above): If yes, the odds ratio increases 2.022 or more than a 2.0 fold increased risk of developing diabetes over a 10 year period. If no, the odds ratio is 0.0.

6) Triglyceride (TG6 above): For every 1 mg/dl increase > 110.0 mg/dl, the odds ratio increases 1.005 and decreases by the same amount for every 1 mg/dl decrease < 110.0 mg/dl.

7) Statin Therapy (CHOLRX6 above): If yes, the odds ratio increases 1.595.

The following parameters were eliminated from the model after stepwise regression analysis: age; gender; CRP; log CRP; HDL-C; history of hypertension; treatment for Gout; Insulin level; log insulin level; homestasis model assessment of insulin resistance (HOMA-IR); log HOMA-IR. The final equation along with a summary of the statistical analysis is provided in Table 3 below.

**Table 3. Logistic Regression Model for Diabetes Prediction**

| Subject Variables | Beta Estimate | Odds Ratio/Unit Change (p value) |
|---|---|---|
| Intercept: -30.00 | | |
| Fasting Glucose | 0.1543 (0.013) | 1.167 (<.0.0001) |
| Body Mass Index | 0.0905 (0.021) | 1.095 (<0.0001) |
| Log Glycated Albumin | 3.7138 (1.004) | 41.01 (0.0002) |
| Log Adiponectin | -0.7934 (0.242) | 0.4523 (0.0011) |
| Parental History of Diabetes | 0.6940 (0.223) | 2.002 (0.0018) |
| Log Triglycerides | 0.0037 (0.001) | 1.004 (0.0047) |
| Statin Therapy | 0.4669 (0.2741) | 1.595 (0.0885) |

All Other Variables Tested were not significant

Final C Statistic 0.916

Equation  X = -30.0 + (glucose X 0.1543) + (BMI X 0.0905) + (log glycated albumin X 3.7138) + (log adiponectin X -0.7934) + (parental history of diabetes X 0.6940) + (log triglycerides X 0.0037) + (statin therapy X 0.4669).

Where 1) fasting glucose is in mg/dL, 2) body mass index is in kg/m$^2$, 3) Glycated Albumin is in %, 4) Adiponectin is in mg/L, 5) Parental History of Diabetes 1= yes, 0 = no, 6) fasting triglycerides in mg/dL, and 7) statin therapy 1= yes, 0 = no.

[0089] Where Probability of developing diabetes over 8.5 years = $1/\exp^{\log X + 1}$

In conclusion, the prior risk assessment models for predicting diabetes described in Example 1 have C statistics of up to 0.85 and have evaluated risk over 4-8 years. The exemplary prediction model of the invention is based on a population of 2,620 men and women with an average age of 58 years, followed for 8.5 years, in which 186 subjects (7.1% of the study population) developed diabetes. The final data is based on 1,823 subjects that did not become diabetic over 8.5 years as compared to 148 people who became diabetic over the same time period. Again, all subjects at baseline did not have diabetes. High risk of developing diabetes over 8.5 years was defined at> 15%, moderate risk defined as 5-15%, and low risk defined as <5%. The exemplary model has a cumulative C statistic of 0.916. Although numerous parameters were evaluated, the following seven were found to be statistically significant in a multivariate model for predicting the development of diabetes mellitus: fasting serum glucose level; body mass index; log plasma percent glycated albumin; log plasma adiponectin; parental history of diabetes; log fasting plasma triglyceride levels; and use of statin therapy. The following factors were also assessed but deemed not significant: age; gender; C reactive protein (CRP) or log CRP; high density lipoprotein cholesterol (HDL-C); hypertension; gout or treatment of gout; serum insulin levels or log serum insulin; homeostasis model assessment of insulin resistance (HOMA-IR, a value calculated from glucose and insulin levels), or log HOMA-IR. The model also controls for cholesterol lowering medications, i.e., statins, which have been implicated in onset of diabetes. The exemplary model provides a significantly better method of predicting diabetes in the general population when compared to other reported methods having C statistic values of 0.85 or less.

The invention may be embodied in other specific forms. The foregoing embodiments are therefore to be considered in all respects illustrative rather than limiting on the invention described herein. Scope of the invention is thus indicated by the appended claims rather than by the foregoing description.

**Claims**

1. A method of predicting the risk of developing type 2 diabetes, the method comprising:

   conducting an assay on a sample from a patient who presents as negative for diabetes to obtain log percent glycated albumin of total albumin;
   obtaining levels of fasting glucose, log adiponectin, and log triglyceride for the patient; and
   determining body mass index (BMI), parental history of diabetes as obtained by asking the patient, and history of statin use for the patient as obtained by asking the patient; and
   predicting development of type 2 diabetes by the patient using a multivariate model including only log percent glycated albumin of total albumin, fasting glucose, log adiponectin, log triglyceride levels, BMI, history of statin use by assigning a value of one if the patient has a history of statin use and a value of zero if the patient does not have a history with statin use, and parental history of diabetes by assigning a value of one if the patient has a parental history of diabetes and a value of zero if the patient does not have a parental history of diabetes.

2. The method according to claim 1, wherein the sample is selected from a group consisting of urine, seminal fluid, saliva, sputum, stool, tissue, and blood.

3. The method according to claim 1, wherein the assay is selected from a group consisting of an enzymatic assay, high-performance liquid chromatography, affinity chromatography, an immunoassay, a radioassay, an enzyme-linked immunosorbent assay, colorimetry, an electrochemical assay, and a combination thereof.

**Patentansprüche**

1. Verfahren zur Vorhersage des Risikos, an Typ-2-Diabetes zu erkranken, wobei das Verfahren umfasst:

   Durchführen eines Tests an einer Probe eines Patienten, der negativ auf Diabetes getestet wurde, zur Ermittlung des logarithmisch transformierten Prozentwertes des glykierten Albumins am Gesamtalbumin;
   Ermitteln des Nüchternglukosespiegels, des logarithmisch transformierten Adiponectinwertes und des logarithmisch transformierten Triglyceridwertes des Patienten; sowie
   Bestimmen des Body Mass Index (BMI), des familiären Diabetes-Hintergrundes durch Befragung des Patienten und der bisherigen Einnahme von Statin durch den Patienten mittels Befragung des Patienten; sowie
   Vorhersagen der Entwicklung von Typ-2-Diabetes durch den Patienten unter Anwendung eines multivariaten Modells, ausschließlich basierend auf dem logarithmisch transformierten Prozentwert des glykierten Albumins am Gesamtalbumin, dem Nüchternglukosespiegel, dem logarithmisch transformierten Adiponectinwert und dem logarithmisch transformierten Triglyceridwert des Patienten, dem BMI, der bisherigen Einnahme von Statin durch Zuordnung eines Wertes von 1 bei erfolgter Einnahme von Statin und eines Wertes von 0 bei bisher nicht erfolgter Einnahme von Statin, sowie des familiären Diabetes-Hintergrundes durch Zuordnung eines Wertes von 1, wenn es eine familiäre Vorbelastung für Diabetes gibt, und eines Wertes von 0, wenn es keine familiäre Vorbelastung für Diabetes gibt.

2. Verfahren nach Anspruch 1, wobei die Probe aus einer Gruppe bestehend aus Urin, Samenflüssigkeit, Speichel, Sputum, Fäzes, Gewebe und Blut ausgewählt wird.

3. Verfahren nach Anspruch 1, wobei der Test aus einer Gruppe bestehend aus enzymatischen Tests, Hochleistungsflüssigkeitschromatographie, Affinitätschromatographie, Immunoassay, Radioassay, Enzyme-linked Immunosorbent Assay, Kolorimetrie, elektrochemischem Assay oder einer Kombination daraus ausgewählt wird.

**Revendications**

1. Procédé de prédiction du risque de développer un diabète de type 2, le procédé comprenant :

la réalisation d'un essai sur un échantillon provenant d'un patient qui se présente comme négatif au diabète pour obtenir un pourcentage logarithmique de l'albumine glyquée de l'albumine totale ;
l'obtention de glycémie à jeun, du logarithme de l'adiponectine et du logarithme de triglycéride pour le patient ; et
la détermination de l'indice de masse corporelle (IMC), des antécédents parentaux de diabète tels qu'obtenus par questionnement du patient et des antécédents de l'utilisation de statine pour le patient tels qu'obtenus par questionnement du patient ; et
la prédiction du développement du diabète de type 2 par le patient grâce à un modèle multivariable comprenant uniquement le pourcentage logarithmique de l'albumine glyquée de l'alumine totale, la glycémie à jeun, le logarithme de l'adiponectine, le logarithme des niveaux de triglycéride, l'IMC, les antécédents d'utilisation de statine par l'attribution d'une valeur unitaire si le patient présente des antécédents d'utilisation de statine et d'une valeur nulle si le patient ne présente pas d'antécédent parental de diabète.

2. Procédé selon la revendication 1, dans lequel l'échantillon est choisi dans l'ensemble constitué d'urine, de fluide séminal, de salive, de crachats, de selles, de tissu et de sang.

3. Procédé selon la revendication 1, dans lequel l'essai est choisi dans un ensemble constitué d'un essai enzymatique, d'une chromatographie en phase liquide à hautes performances, d'une chromatographie d'affinité, d'un immuno-essai, d'un radio-essai, d'une méthode ELISA, de colorimétrie, d'un essai électrochimique et d'une combinaison de ceux-ci.

Computer
249

I/O 154

Processor
159

Memory
163

100

Network
109

Server 113

Data File
117

Processor
121

Interface
Module 125

Memory
129

Terminal
267

I/O 171

Processor
175

Memory
179

**Figure 1**

Figure 2

300

```
┌──────────────────┐
│      MODEL       │
│   DEVELOPMENT    │
└──────────────────┘
         │
         ▼
```

301

```
┌──────────────────┐
│      OBTAIN      │
│  REPRESENTATIVE  │
│    POPULATION    │
│      DATA        │
└──────────────────┘
         │
         ▼
```

302

```
┌──────────────────┐
│     PREPARE      │
│    POPULATION    │
│    DATA SET      │
└──────────────────┘
         │
         ▼
```

303

```
┌────────────────────────────┐
│    PARAMETER SELECTION      │
└────────────────────────────┘
         │
         ▼
```

304

```
┌──────────────────┐
│      MODEL        │
│  SELECTION AND    │
│   VALIDATION      │
└──────────────────┘
         │
         ▼
```

```
┌──────────────────┐
│      MODEL        │
│   APPLICATION     │
└──────────────────┘
```

**Figure 3**

400

```
┌──────────────────┐
│ MODEL APPLICATION │
└──────────────────┘
         │
         ▼
```

401

```
┌──────────────────────────────┐
│  OBTAIN SUBJECT PARAMETER DATA │
└──────────────────────────────┘
         │
         ▼
```

402

```
┌──────────────────────────────┐
│   PREPARE SUBJECT DATA SET     │
└──────────────────────────────┘
         │
         ▼
```

403

```
┌──────────────────────────────┐
│  INPUT SUBJECT DATA INTO MODEL │
└──────────────────────────────┘
         │
         ▼
```

404

```
┌──────────────────────────────┐
│      OUTPUT INDEX VALUE        │
└──────────────────────────────┘
         │
         ▼
```

```
┌──────────────────────────────┐
│      RISK DETERMINATION        │
└──────────────────────────────┘
```

**Figure 4**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 7659107 B
- US 7871789 B
- US 20070015291 A
- US 20090246801 A
- US 20100167306 A
- US 20100204557 A

- US 7608405 B
- US 8026345 B
- US 3703591 A
- US 4245041 A
- US 4999289 A

### Non-patent literature cited in the description

- **D'AGOSTINO et al.** *JAMA,* 2001, vol. 286, 180-187
- **STERN, M. P. et al.** *Am. J. Epidemiol.,* 1984, vol. 120, 834-851
- **STERN, M. P. et al.** *Diabetes,* 1993, vol. 42, 706-714
- **BURKE, J. P et al.** *Arch. Intern. Med.,* 1999, vol. 159, 1450-1456

- **WILSON et al.** *Arch Intern Med.,* 28 May 2007, vol. 167 (10), 1068-74
- **SCHMIDT et al.** *Diabetes Care,* August 2005, vol. 28 (8), 2013-8
- **MANN et al.** *Am J Epidemiol.,* 01 May 2010, vol. 171 (9), 980-8
- **KOLBERG et al.** *Diabetes Care.,* July 2009, vol. 32 (7), 1207-12